# EUROPEAN PATENT APPLICATION

(11) **EP 3 391 909 A1**
(43) Date of publication of application: **24.10.2018**
(21) Application number: 17167090.4
(22) Date of filing: 19.04.2017
(51) Int. Cl.: A61K 47/24, A61K 47/28, A61K 9/10, A61K 9/107, A61K 31/122

(54) **AQUEOUS SUSPENSION COMPRISING MIXED MICELLES LOADED WITH A LIPOPHILIC PHYSIOLOGICALLY ACTIVE SUBSTANCE**

(71) Applicant: Tiofarma B.V., 3261 LW Oud-Beijerland (NL)
(72) Inventor: ROOIMANS, Thijs, 3021 AG Rotterdam (NL)
(74) Representative: Nederlandsch Octrooibureau

(57) **Abstract**

The invention provides an aqueous suspension of mixed micelles loaded with a lipophilic physiologically active substance, the mixed micelles comprising:
(i) a phospholipid, and
(ii) a taurine conjugate of a bile acid.

The mixed micelles of the present invention exhibit very high stability at low pH.

## Description

### TECHNICAL FIELD OF THE INVENTION

The present invention relates to an aqueous suspension of mixed micelles loaded with a lipophilic physiologically active substance, the mixed micelles comprising (i) a phospholipid, and (ii) a taurine conjugate of a bile acid.

The aqueous suspension of the present invention is particularly suitable for oral administration of lipophilic vitamins and drugs.

### BACKGROUND OF THE INVENTION

A micelle is an aggregate (or supramolecular assembly) of surfactant molecules dispersed in a liquid colloid. A typical micelle in aqueous solution forms an aggregate with the hydrophilic "head" regions in contact with surrounding solvent, sequestering the hydrophobic "tail" regions in the micelle centre. Micelles are usually spherical in shape. Other shapes such as ellipsoids, cylinders and discs are also possible. The shape and size of a micelle are a function of the molecular geometry of its surfactant molecules and solution conditions such as surfactant concentration, temperature, pH, and ionic strength.

Micelles are known to have an anisotropic water distribution within their structure. In other words, the water concentration decreases from the surface towards the core of the micelle, with a completely hydrophobic (water-excluded) core. Consequently, the spatial position of a solubilized substance in a micelle will depend on its polarity: nonpolar molecules will be solubilized in the micellar core, and substances with intermediate polarity will be distributed along the surfactant molecules in certain intermediate positions.

An important property of micelles that has particular significance in pharmacy is their ability to increase the solubility of lipophilic substances in water and to thereby increase their bioavailability. In this context, solubilisation can be defined as the spontaneous dissolving of a substance by reversible interaction with the micelles of a surfactant in water to form a thermodynamically stable isotropic solution with reduced thermodynamic activity of the solubilized material. The solubility of a lipophilic substance in aqueous liquid as a function of the concentration of a micelle forming surfactant is very low until the surfactant concentration reaches its critical micelle concentration (CMC). At surfactant concentrations above the CMC the solubility usually increases linearly with the concentration of surfactant, indicating that solubilisation is related to micellisation.

Individuals with cholestatic liver diseases produce or secrete insufficient bile. Bile contains high amounts of bile salts and phosphatidylcholine, which allows mixed micelle formation. Mixed micelles are composed of bile acids, (lyso)phospholipids, fatty acids, mono- di- or triglycerides, cholesterol and - when present - lipophilic molecules such as phylloquinone (vitamin K₁). The lack of bile is generally believed to be the main reason for poor absorption of fat soluble vitamins in cholestatic subjects. Patients with cholestasis need periodic administration of phylloquinone, amongst others.

Phylloquinone is one of the four fat soluble vitamins (vitamin A, D, E and K). Under normal conditions, the majority of phylloquinone is absorbed from the jejunum and ileum, and only a small percentage from the colon. The route of absorption is similar to that of many dietary lipids. This process includes solubilisation into mixed micelles, uptake by enterocytes, packing into chylomicrons and subsequent exocytosis into the lymphatic system.

Undiagnosed phylloquinone deficiency is of particular concern for newborns, as a deficiency may result in spontaneous life threatening haemorrhages. Three types of vitamin K deficiency bleedings (VKDB) are described in literature, the classification is solely based on the time of bleeding after birth. An early VKDB happens on the first day of life, the classical VKDB shortly (day 1 to 7) after birth and the late VKDB occurs after the first week of life (>1 week; ≤6 months). The underlying mechanism is different for each VKDB. Late VKDB is most severe and mostly caused by malabsorption of phylloquinone by an underlying cholestatic liver disease, instead of insufficient 'intake' by the mother (early VKDB) or newborn (classical VKDB). More than 60% of infants with a late VKDB present with a sudden and unpredictable acute intracranial haemorrhage. The mortality is almost 15% and at least 40% of the survivors will suffer long-term neurological handicaps.

VKDB can easily be prevented by sufficient phylloquinone supplementation, although there is still an ongoing debate about the ideal dosing regimen. Classical phylloquinone droplets in oil are unsuited for the supplementation of phylloquinone to cholestatic infants. Ideally, a new pharmaceutical formulation would mimic the physiological conditions by presenting phylloquinone in mixed micelles. This approach has been explored for phylloquinone, which is available as Konakion^{®} MM (Roche, Basel, Switzerland). Here, phylloquinone is incorporated into mixed micelles composed of glycocholic acid and phosphatidylcholine. Initial reports were positive showing high serum levels after oral dosing, later reports showed that the mixed micellar formulation of Roche gives low serum phylloquinone levels and does not improve efficacy of oral phylloquinone prophylaxis (Pereira et al., Intestinal absorption of mixed micellar phylloquinone (vitamin K1) is unreliable in infants with conjugated hyperbilirubinaemia: implications for oral prophylaxis of vitamin K deficiency bleeding. Arch Dis Child Fetal Neonatal Ed. 2003;88:113-8.).

Sun et al. (A Mixed Micelle Formulation for Oral Delivery of Vitamin K, Pharm Res (2016) 33:2168-2179) describe a study that aimed to develop a stable micellar formulation of vitamin K for oral delivery, because the commercial and clinically used formulation of vitamin K (Konakion^{®} MM) destabilizes at gastric pH resulting in low bioavailability of this vitamin in neonates with cholestasis. Mixed micelles composed of egg phosphatidylcholine, 1,2-distearoyl-sn-glycero-3-phosphoethanolamine-N-[methoxy(polyethyleneglycol)-2000] and glycocholic acid, with and without vitamin K, were prepared by a film hydration method. The mixed micelles so prepared were found to have superior stability at low pH in comparison to Konakion^{®} MM.

### SUMMARY OF THE INVENTION

The present inventors have developed a mixed micelle formulation for the oral delivery of lipophilic physiologically active substances that exhibit very high stability at low pH. The mixed micelles employed in accordance with the present invention comprise a surfactant system that is composed of at least (i) a phospholipid and (ii) a taurine conjugate of a bile acid.

Thus, a first aspect of the invention relates to an aqueous suspension of mixed micelles loaded with a lipophilic physiologically active substance, the mixed micelles comprising:
(i) a phospholipid; and
(ii) a taurine conjugate of a bile acid.

Tests conducted by the inventors have shown that the mixed micelles of the present invention are very stable at low pH. In addition, unlike the mixed micelles described in the aforementioned article by Sun et al., the mixed micelles of the present invention do not require the use of expensive components, such as 1,2-distearoyl-sn-glycero-3-phosphoethanolamine-N-[methoxy(polyethyleneglycol)-2000].

Another aspect of the invention relates to the use of the aforementioned aqueous suspension in the treatment of a disease, said treatment comprising administration of the aqueous suspension.

### DETAILED DESCRIPTION OF THE INVENTION

The present invention provides an aqueous suspension of mixed micelles loaded with a lipophilic physiologically active substance, the mixed micelles comprising:
(i) a phospholipid, and
(ii) a taurine conjugate of a bile acid.

The term "mixed micelles" as used herein refers to aggregates of surfactant molecules that include at least the two aforementioned surfactants (phospholipid and taurine conjugate of bile acid), which aggregates are dispersed in the aqueous suspension.

The terminology "mixed micelles loaded with a lipophilic physiologically active substance" means that this substance is contained within the mixed micelles.

The term "physiologically active substance" as used herein refers to a substance that can exhibit *in vivo* physiological activity in a mammal. Examples of physiologically active substances include vitamins and drugs.

The term "phospholipid" as used herein, unless indicated otherwise, refers to compounds derived from fatty acids and a phosphate-containing compound attached to glycerol or the amino alcohol sphingosine. Examples of phospholipids are phosphatidylcholine, phosphatidylinositol, phosphatidylethanolamine and phosphatidic acid.

The term "non-ionic amphiphilic surfactant" as used herein refers to organic compounds that contain both hydrophobic groups and hydrophilic groups and wherein the hydrophilic group does not carry a charge.

The term "treating" as used herein comprises both therapeutic and prophylactic treatment.

The mixed micelles in the aqueous suspension typically have a volume weighted average diameter of less than 50 nm, more preferably of less than 30 nm and most preferably in the range of 3 to 15 nm.

The lipophilic physiologically active substance that is contained in the mixed micelles of the present suspension typically has a solubility in water at 20°C of less than 1 mg/ml. More preferably, this lipophilic substance has a solubility in water at 20°C of less than 0.1 mg/ml, most preferably of less than 0.01 mg/ml.

In accordance with a preferred embodiment of the invention, the suspension contains at least 0.1 mg, more preferably at least 1.0 mg and most preferably 10-1,000 mg of the lipophilic physiologically active substance per 100 ml.

According to a particularly preferred embodiment the lipophilic physiologically active substance is selected from vitamin K, vitamin A, vitamin D, vitamin E and combinations thereof. Even more preferably, said lipophilic substance is vitamin K, most preferably vitamin K₁ (phylloquinone).

According to a particularly preferred embodiment, the aqueous suspension contains at least 1 mg vitamin K₁ per 100 ml, more preferably at least 10 mg vitamin K₁ per 100 ml, more preferably 50-1,000 mg vitamin K₁ per 100 ml, and most preferably 70-300 mg vitamin K₁ per 100 ml.

The phospholipid in the mixed micelle is preferably selected from phosphatidylcholine, phosphatidylinositol, phosphatidylethanolamine, phosphatidic acid and combinations thereof. Most preferably, the phospholipid is phosphatidylcholine.

The aqueous suspension of the present invention preferably contains 1-40 g phospholipid per 100 ml, more preferably 1.2-20 g phospholipid per 100 ml and most preferably 1.5-8 g phospholipid per 100 ml.

According to a particularly preferred embodiment, the suspension contains at least 1 g, more preferably at least 1.2 g and most preferably at least 1.5 g phosphatidylcholine per 100 ml.

The taurine conjugate of a bile acid employed in accordance with the present invention preferably is a taurine conjugate of a bile acid selected from cholic acid, deoxycholic acid, chenodeoxycholic acid, glycochenodeoxycholic acid, lithocholic acid and combinations thereof. Most preferably, the taurine conjugate is a taurine conjugate of cholic acid (taurocholic acid).

The aqueous suspension preferably contains 0.5-40 g, more preferably 1-20 g and most preferably 2-15 g of the taurine conjugate of a bile acid per 100 ml.

According to a particularly preferred embodiment the suspension contains at least 0.5 g, more preferably at least 1 g and most preferably at least 2 g taurocholic acid per 100 ml.

The suspension preferably contains not more than a limited amount of a glycine conjugate of a bile acid, such as glycocholic acid. Taurine conjugates of a bile acid and glycine conjugates of a bile acid are preferably present in the aqueous suspension in a molar ratio of at least 2:1, more preferably of at least 3:1 and most preferably of at least 5:1.

Typically, the suspension contains not more than 5 g, more preferably not more than 1 g and most preferably not more than 0.5 g glycocholic acid per 100 ml.

The aqueous suspension of the present invention preferably contains the phospholipid and the taurine conjugate of a bile acid in a weight ratio that lies in the range of 1:10 to 10:1, more preferably in the range of 1:5 to 5:1, most preferably in the range of 1:4 to 2:1.

According to a particularly preferred embodiment of the invention, the mixed micelles of the aqueous suspension further comprise a non-ionic amphiphilic surfactant.

The non-ionic amphiphilic surfactant that is present in the mixed micelles preferably has a HLB value in the range of 9 to 20, more preferably in the range of 11 to 15.

Preferably, the non-ionic amphiphilic surfactant is selected from D-α-tocopheryl polyethylene glycol succinate (TPGS), sucrose monoester of a fatty acid, polysorbate, macrogolglycerol ricinoleate, poly(ethylene glycol)-block-poly(propylene glycol)-block-poly(ethylene glycol) and combinations thereof. Most preferably, the non-ionic amphiphilic surfactant is TPGS.

The aqueous suspension preferably contains 0.2-50 g, more preferably 0.5-25 g, most preferably 1-15 g of the non-ionic amphiphilic surfactant per 100 ml.

According to particularly preferred embodiment, the suspension contains at least 0.2 g, more preferably at least 0.5 g and most preferably at least 1 g of D-α-tocopheryl polyethylene glycol succinate (TPGS) per 100 ml.

In accordance with another preferred embodiment, the suspension contains the taurine conjugate of a bile acid and the non-ionic amphiphilic surfactant in a weight ratio that lies in the range of 1:10 to 30:1, more preferably in the range of 1:5 to 15:1, most preferably in the range of 1:2 to 10:1.

The lipophilic physiologically active substance is preferably present in the aqueous suspension in a concentration of at least 0.05%, more preferably at least 0.1 % and most preferably 0.3-5.0% calculated by weight of the combined amount of the phospholipid, the taurine conjugate of a bile acid and the non-ionic amphiphilic surfactant that is present in the suspension.

The aqueous suspension according to the present invention typically has a water content of 50-95 wt.%. More preferably, the suspension has a water content of 70-90 wt.%, most preferably of 75-85 wt.%.

Another aspect of the present invention relates to the use of the aqueous suspension as described herein in the treatment of a disease, said treatment comprising administration of the suspension.

The aforementioned treatment preferably comprises oral, intravenous or intramuscular administration of the suspension. Most preferably, the treatment comprises oral administration of the suspension.

In accordance with a particularly preferred embodiment of the invention, the lipophilic physiologically active substance contained in mixed micelles is vitamin K₁ and the suspension is used for the treatment of vitamin K deficiency, more preferably prophylactic treatment of vitamin K deficiency.

The aqueous suspension of the present invention is particularly suitable for use in the treatment, particularly prophylactic treatment of vitamin deficiency in a subject suffering from cholestasis, especially for the prophylactic treatment of vitamin K deficiency in infants.

The invention is further illustrated by the following non-limiting examples.

### EXAMPLES

### Example 1

Mixed micelles were prepared using a thin film hydration method. Egg phosphatidylcholine and phylloquinone (vitamin K₁) were dissolved in methanol in a round-bottom flask. Organic solvent was evaporated under a vacuum (50 mbar) at 55 °C using a Büchi R-210 Rotavapor^{®} equipped with a V-850 vacuum controller for at least one hour. Residual organic solvent was further evaporated under a gentle stream of high purity nitrogen.

The acquired film was hydrated for at least 4 hours with a solution taurocholic acid in citrate buffer (88.5 mM trisodium citrate dihydrate and 11.5 mM citric acid monohydrate, pH = 6.0) under continuous mixing. The initial temperature was 55 °C after which the solution was allowed to gradually cool down to room temperature. To obtain the final particles the micellar solution was filtered once through a 0.2 µm syringe filter (Sartorius, Goettingen, Germany).

After filtration, uniformly distributed particles were obtained with an average size of 4.1 ± 0.1 nm and a polydispersity index of 0.12 ± 0.03 indicating homogeneous distributed micelles. The micelles were composed of phylloquinone, egg phosphatidylcholine and taurocholic acid in a concentration of 1, 40 and 65 mg/ml, respectively.

### Example 2

Mixed micelles comprising phylloquinone, egg phosphatidylcholine, taurocholic acid and D-α-tocopheryl polyethylene glycol 1000 succinate (TPGS) were prepared according to Example 1, with TGPS being dissolved in methanol together with egg phosphatidylcholine and phylloquinone.

After filtration, uniformly distributed particles were obtained with an average size of 7.2 ± 0.1 nm and a polydispersity index of 0.18 ± 0.01 indicating homogeneous distributed micelles. The micelles were composed of phylloquinone, egg phosphatidylcholine, taurocholic acid and TPGS in a concentration of 1, 40, 65 and 107.5 mg/ml, respectively.

These particles can be freeze dried to produce a lyophilisate. To produce a micellar suspension the lyophilized powder needs to be reconstituted with the appropriate amount of RO-water and passed through a 0.2 µm syringe filter once.

### Example 3

The acidic stability of the mixed micelles of Example 1 and 2 was compared to that of the commercial product Konakion^{®} (mixed micelles comprising phylloquinone (10 mg/ml), egg phosphatidylcholine and glycocholic acid). The mixed micelles of Example 1 were composed of phylloquinone, egg phosphatidylcholine and taurocholic acid in a concentration of 1, 40 and 65 mg/ml, respectively. The mixed micelles of Example 2 were composed of phylloquinone, egg phosphatidylcholine, taurocholic acid and TPGS in a concentration of 1, 40, 65 and 107.5 mg/ml, respectively.

To evaluate the acidic stability the pH was lowered to 1.0 using 1.0 M HCl. Control samples were diluted with the same volume using citrate buffer (pH = 6.0). Particle size and polydispersity index (PDI) were determined using a DLS Malvern CGS-3 goniometer (Malvern Instruments Ltd., Malvern, U.K.) coupled to an LSE-5003 autocorrelator, a thermostated water bath, a He-Ne laser (25 mW, 633 nm, equipped with a model 2500 remote interface controller, Uniphase) and a computer with DLS software (PCS, version 3.0.2.1, Malvern). Measurements were carried out at 25°C with a measurement angle of 90°. The solvent viscosity and refractive index were corrected by the software. The results are shown in Table 1.

**Table 1**

| **Formulation** | **pH** | **Dilution** | **Z-ave (nm)** | **PDI** |
|---|---|---|---|---|
| Example 1 | 6 | 1x | 4.11 ± 0.08 | 0.12 ± 0.03 |
| | 6 | 10x | 5.58 ± 0.15 | 0.17 ± 0.04 |
| | 1 | 10x | 10.26 ± 0.02 | 0.09 ± 0.01 |
| Example 2 | 6 | 1x | 7.18 ± 0.08 | 0.18 ± 0.01 |
| | 6 | 10x | 9.56 ± 0.05 | 0.05 ± 0.03 |
| | 1 | 10x | 9.65 ± 0.06 | 0.03 ± 0.01 |
| Commercial product | 6 | 1x | 2.61 ± 0.02 | 0.68 ± 0.01 |
| | 6 | 10x | 12.44 ± 0.10 | 0.20 ± 0.01 |
| | 1 | 10x | 1885.99 ± 2970.32 | 0.68 ± 0.33 |

Particle size and PDI of the mixed micelles of Example 1 and Example 2 were essentially unaffected by the pH decrease, whereas the commercial formulation showed a sharp increase in particle size upon exposure to acidic environment.

Also, the visual appearance (color and clarity) of the mixed micelles of Examples 1 and 2 remained unchanged upon exposure to acidic environment, indicative of the absence of particulate matter. The commercial formulation, however, became opalescent immediately upon exposure to acidic environment

### Example 4

At different acidic pH's the stability of the mixed micelles of Example 1 and 2 was compared to that of the commercial product Konakion^{®} (mixed micelles comprising phylloquinone (10 mg/ml), egg phosphatidylcholine and glycocholic acid). The mixed micelles of Example 1 were composed of phylloquinone, egg phosphatidylcholine and taurocholic acid in a concentration of 1, 40 and 65 mg/ml, respectively. The mixed micelles of Example 2 were composed of phylloquinone, egg phosphatidylcholine, taurocholic acid and TPGS in a concentration of 1, 40, 65 and 107.5 mg/ml, respectively.

The pH of the micellar suspension was lowered in a stepwise fashion using 1.0 M HCl. The dilution factor (10x) was kept constant for all samples. Particle size and PDI were determined as described under Example 3. The results are shown in Table 2.

**Table 2**

| **pH** | **Example 1** | | **Example 2** | | **Commercial formulation** | |
|---|---|---|---|---|---|---|
| | **Z-ave (nm)** | **PDI** | **Z-ave (nm)** | **PDI** | **Z-ave (nm)** | **PDI** |
| 6 | 5.58 ± 0.15 | 0.17 ± 0.04 | 9.56 ± 0.05 | 0.05 ± 0.03 | 12.44 ± 0.10 | 0.20 ± 0.01 |
| 5 | 7.31 ± 0.12 | 0.08 ± 0.03 | 9.13 ± 0.06 | 0.02 ± 0.02 | 23.09 ± 0.08 | 0.13 ± 0.01 |
| 4 | 7.39 ± 0.09 | 0.06 ± 0.02 | 9.00 ± 0.01 | 0.03 ± 0.01 | 71.54 ± 0.91 | 0.10 ± 0.01 |
| 3 | 7.30 ± 0.10 | 0.09 ± 0.02 | 8.85 ± 0.10 | 0.03 ± 0.02 | 252.00 ± 6.54 | 0.62 ± 0.02 |
| 2 | 7.41 ± 0.08 | 0.10 ± 0.02 | 9.04 ± 0.05 | 0.02 ± 0.01 | ND | ND |
| 1 | 10.26 ± 0.02 | 0.09 ± 0.01 | 9.65 ± 0.06 | 0.03 ± 0.01 | ND | ND |

| | | | | | | |
|---|---|---|---|---|---|---|
| Abbreviations: ND: Not defined. | | | | | | |

Particle size and PDI of the mixed micelles of Example 1 and 2 remained essentially unchanged throughout the pH range of 1-6, whereas the commercial formulation showed a sharp increase in particle size when the pH dropped below 5.

## Claims

1. An aqueous suspension of mixed micelles loaded with a lipophilic physiologically active substance, the mixed micelles comprising:
(i) a phospholipid; and
(ii) a taurine conjugate of a bile acid.

2. Aqueous suspension according to claim 1, wherein the lipophilic physiologically active substance is selected from vitamin K, vitamin A, vitamin D, vitamin E and combinations thereof.

3. Aqueous suspension according to claim 1 or 2, wherein the phospholipid is phosphatidylcholine.

4. Aqueous suspension according to any one of the preceding claims, wherein the taurine conjugate of a bile acid is taurocholic acid.

5. Aqueous suspension according to any one of the preceding claims, wherein the suspension contains at least 0.1 mg of the lipophilic physiologically active substance per 100 ml.

6. Aqueous suspension according to any one of the preceding claims, wherein the suspension contains 1-40 g phospholipid per 100 ml.

7. Aqueous suspension according to any one of the preceding claims, wherein the suspension contains 0.5-40 g of the taurine conjugate of a bile acid per 100 ml.

8. Aqueous suspension according to any one of the preceding claims, wherein the mixed micelles further comprise a non-ionic amphiphilic surfactant.

9. Aqueous suspension according to claim 8, wherein the non-ionic amphiphilic surfactant has a HLB value in the range of 9 to 20.

10. Aqueous suspension according to claim 8 or 9, wherein the non-ionic amphiphilic surfactant is D-α-tocopheryl polyethylene glycol succinate (TPGS).

11. Aqueous suspension according to any one of claims 8-10, wherein the suspension contains 0.2-50 g of the non-ionic amphiphilic surfactant per 100 ml.

12. Aqueous suspension according to any one of the preceding claims, wherein the suspension has a water content of 50-95 wt.%.

13. Aqueous suspension according to any of the preceding claims for use in the treatment of a disease, said treatment comprising administration of the suspension.

14. Aqueous suspension for use in the treatment of a disease according to claim 13, said use comprising oral administration of the suspension.

15. Aqueous suspension for use in the treatment according to claim 13 or 14, wherein the lipophilic physiologically active substance contained in the suspension is vitamin K₁, and wherein the suspension is used for the treatment of vitamin K deficiency.
